(19) Europäisches Patentamt
European Patent Office
Office européen des brevets

(11) Veröffentlichungsnummer : **0 282 502 B1**

(12) # EUROPÄISCHE PATENTSCHRIFT

(45) Veröffentlichungstag der Patentschrift : **15.05.91 Patentblatt 91/20**

(51) Int. Cl.$^5$ : **C07D 233/90**, C07D 413/04, A61K 31/415, A61K 31/41

(21) Anmeldenummer : **87904844.5**

(22) Anmeldetag : **30.07.87**

(86) Internationale Anmeldenummer : **PCT/DE87/00342**

(87) Internationale Veröffentlichungsnummer : **WO 88/01268 25.02.88 Gazette 88/05**

(54) **IMIDAZOL-DERIVATE.**

Die Akte enthält technische Angaben, die nach dem Eingang der Anmeldung eingereicht wurden und die nicht in dieser Patentschrift enthalten sind.

(30) Priorität : **11.08.86 DE 3627155**

(43) Veröffentlichungstag der Anmeldung : **21.09.88 Patentblatt 88/38**

(45) Bekanntmachung des Hinweises auf die Patenterteilung : **15.05.91 Patentblatt 91/20**

(84) Benannte Vertragsstaaten : **AT BE CH DE FR GB IT LI LU NL SE**

(56) Entgegenhaltungen :
**EP-A- 0 113 570**
**EP-A- 0 185 961**
**EP-A- 0 199 206**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **BIERE, Helmut**
**Zeltinger Str. 15**
**W-1000 Berlin 28 (DE)**
Erfinder : **HUTH, Andreas**
**Kirchweg 55**
**W-1000 Berlin 38 (DE)**
Erfinder : **RAHTZ, Dieter**
**Krottnaurer Str. 24a**
**W-1000 Berlin 38 (DE)**
Erfinder : **SCHMIECHEN, Ralph**
**Bayernring 27**
**W-1000 Berlin 42 (DE)**
Erfinder : **SEIDELMANN, Dieter**
**Stierstr. 14**
**W-1000 Berlin 41 (DE)**
Erfinder : **SCHNEIDER, Herbert, Hans**
**Duisburger Str. 20**
**W-1000 Berlin 15 (DE)**
Erfinder : **STEPHENS, David, Norman**
**Hildegardstr. 16a**
**W-1000 Berlin 31 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 282 502 B1

## Beschreibung

Die Erfindung betrifft neue ZN-aktive Imidazolderivate der allgemeinen Formel I

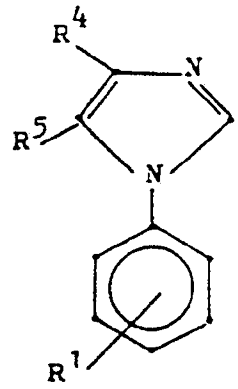

(I),

worin

$R^1$ Wasserstoff oder Halogen in o-, m- oder p-Stellung, wobei das Halogen einfach oder mehrfach am Phenylrest auftreten kann,

$R^4$ $-COOR^6$, 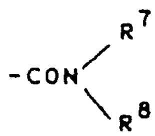 , $-CN$,

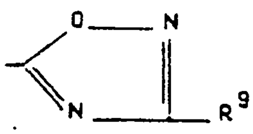

oder

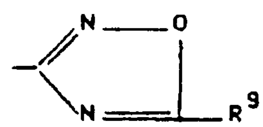

mit $R^6$ und $R^9$ in der Bedeutung von Wasserstoff oder einer geraden oder verzweigten Alkylgruppe mit 1 bis 6 Kohlenstoffatomen, $R^7$ und $R^8$ sind gleich oder verschieden und stellen Wasserstoff oder eine gerade oder verzweigte Alkylgruppe mit 1 bis 6 Kohlenstoffatomen dar oder $R^7$ und $R^8$ stellen gemeinsam mit dem Stickstoffatom einen gegebenenfalls ein weiteres Heteroatom enthaltenden gesättigten heterocyclischen Fünf- oder Sechsring dar und

$R^5$ Wasserstoff, eine Alkylgruppe mit 1 bis 6 Kohlenstoffatomen oder eine Alkoxyalkylgruppe mit 1 bis 6 Kohlenstoffatomen bedeuten.

Unter Halogen ist Fluor, Chlor, Brom oder Jod zu verstehen, wobei Fluor und Chlor bevorzugt sind.

Unter Alkyl ist jeweils eine gerade oder verzweigte Alkylgruppe mit 1 bis 6 Kohlenstoffatomen zu verstehen, beispielsweise die Methyl-, Ethyl-, Propyl-, Isopropyl-, Butyl-, Pentyl- und Hexylgruppe. Als bevorzugte Ausführungsform für $R^5$ in der Bedeutung Alkoxyalkyl ist $C_{1-4}$ Alkoxy-$C_{1-4}$ Alkyl, insbesondere $C_{1-4}$ Alkoxymethyl anzusehen.

Wenn $R^7$ und $R^8$ gemeinsam mit dem Stickstoffatom einen gegebenenfalls ein weiteres Heteroatom enthaltenden gesättigten heterocyclischen Fünf- oder Sechsring darstellen, steht

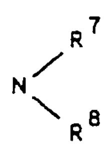

beispielsweise für Pyrrolidin, Piperidin, Morpholin oder Piperazin.

Es ist bekannt, daß bestimmte Stellen im zentralen Nervensystem von Vertebraten eine hohe spezifische Affinität für die Bindung von 1,4- und 1,5-Benzodiazepinen aufweisen (Squires, R. F. und Braestrup, C., Nature (London) 266 (1977) 734). Die Stellen werden Benzodiazepin-Rezeptoren genannt.

Es wurde gefunden, daß die erfindungsgemäßen substituierten Imidazolderivate, obwohl sie sich in ihrer chemischen Struktur von den Benzodiazepinen stark unterscheiden, überraschenderweise eine starke Affinität und Spezifität für die Bindung an die Benzodiazepin-Rezeptoren zeigen und gleichzeitig nur geringe Toxizität aufweisen.

So können die erfindungsgemäßen Verbindungen beispielsweise auf die von Benzodiazepinen bekannten Eigenschaften agonistische, invers agonistische und antagonistische Effekte ausüben.

Benzodiazepine entfalten beispielsweise anticonvulsive, anxiolytische und muskelrelaxierende sowie sedierende Effekte.

Ein Nachteil der Benzodiazepine ist das relativ breite Wirkungsspektrum mit geringer Selektivität.

Es besteht daher nach wie vor ein Bedürfnis an gut vertraglichen Substanzen mit dissozierter ZN-Wirksamkeit.

Die erfindungsgemäßen Verbindungen erscheinen aufgrund ihrer biologischen Wirksamkeit als Psychopharmaka für die Humanmedizin geeignet. Sie können zu psychopharmazeutischen Präparationen, zum Beispiel für die orale und parenterale Anwendung, formuliert werden.

Als Formulierungshilfsstoffe sind physiologisch verträgliche organische und anorganische Trägersubstanzen geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsaure-mono- und -diglyceride, Pentaerythritolfettsaureester, Hydroxymethylcellulose und Polyvinylpyrrolidon.

Die pharmazeutischen Präparationen können sterilisiert und/oder mit Hilfsstoffen, wie Schmiermittel, Konservierungsstoffen, Stabilisatoren, Hetzmittel, Emulgatoren, Puffermittel und Farbstoffen, versetzt werden.

Für die parenterale Anwendung sind insbesondere Injektionslosungen oder Suspensionen, insbesondere waßrige Lösungen der aktiven Verbindungen in polyhydroxyethoxyliertem Rizinusöl, geeignet. Als Tragersysteme können auch grenzflächenaktive Hilfsstoffe wie Salze der Gallensauren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder einem Kohlenwasserstoffträger oder -binder, wie zum Beispiel Lactose, Hais- oder Kartoffelstarke, geeignet. Die Anwendung kann auch in flüssiger Form erfolgen, wie zum Beispiel als Saft, dem gegebenenfalls ein Sußstoff beigefugt wird.

Die erfindungsgemäßen Verbindungen werden in einer Dosiseinheit von 0,05 bis 10 mg aktiver Substanz in einem physiologisch vertraglichen Träger eingebracht.

Die erfindungsgemaßen Verbindungen werden in einer Dosis von 0,1 bis 300 mg/Tag, vorzugsweise 1-30 mg/Tag, angewendet.

Die Herstellung der erfindungsgemäßen Verbindungen der allgemeinen Formel I erfolgt dadurch, daß man ein Anilin der allgemeinen Formel II

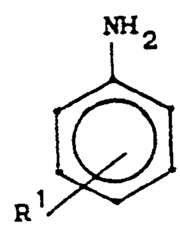

(II),

worin

$R^1$ die in Formel I angegebene Bedeutung hat, mit einem 2-Azabutadien der allgemeinen Formel III

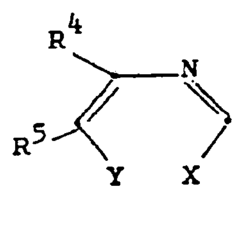

(III),

worin

$R^4$ und $R^5$ die in Formel I angegebene Bedeutung haben und

X und Y Fluchtgruppen darstellen,

in Gegenwart von Säuren bei Temperaturen von 0 bis 150°C umsetzt und gegebenenfalls eine im Molekül vorhandene Estergruppe umestert oder verseift, eine freie Carboxylgruppe gegebenenfalls verestert, amidiert oder mit einem Amidoxim der Formel $R^9$-C(=NOH)$NH_2$ zum 5-Oxadiazolylderivat umsetzt und gegebenenfalls eine im Molekül vorhandene Nitrilgruppe zur Carbonylamid- oder Carboxylgruppe hydrolisiert oder über die Iminoestergruppe in die Estergruppe ($COOR^6$) oder mit Hydroxylamin über das Amidoxim und anschließend mit einer Alkancarbonsäure der Formel $R^9$-COOH oder einem aktivierten Derivat der Säure in das 3-Oxadiazolylderivat überführt.

Die erfindungsgemäße Umsetzung von Anilinen der Formel II mit 2-Azabutadienen der Formel III zu den Imidazolderivaten der Formel I erfolgt in Gegenwart von Säuren bei Temperaturen von 0 bis 150°C. Als Fluchtgruppen X und Y kommen insbesondere Dialkylamine, wie Dimethyl- und Diethylamin, und cyclische Amine, wie Pyrrolidin, infrage.

Die Umsetzung wird beispielsweise so ausgeführt, daß das Anilinderivat und das Azabutadien in einer organischen Säure, wie zum Beispiel Ameisensäure, Essigsäure, Propionsäure oder Trifluoressigsäure, zunächst bei Raumtemperatur gerührt und dann bis zur Siedetemperatur des Reaktionsgemisches (bis etwa 120°C) erhitzt wird.

Die Säure kann gleichzeitig als Reaktionsmittel und auch als Lösungsmittel dienen. Es können aber auch Lösungsmittel-wie zum Beispiel Alkohole, Ether, Ketone, Ester, wie Ethylacetat, Kohlenwasserstoffe, wie Toluol, oder Halogenkohlenwasserstoffe, wie Tetrachlorkohlenstoff, zugesetzt werden.

Die Menge der Säure kann in weiten Grenzen variiert werden, sie wird jedoch im Überschuß angewendet. Vorzugsweise wird ein 3-10 facher Säureüberschuß, bezogen auf das Anilin und daß Azabutadien, gewählt.

Die Molverhältnisse von Anilin und Azabutadien sind für den Erfolg der Umsetzung nicht kritisch. Im allgemeinen wird man etwa gleiche molare Mengen der Reaktionspartner einsetzen, wobei molare Mengen von 1 Anilin und 1,1 Azabutadien bevorzugt sind. Die erfindungsgemäße Umsetzung kann grundsätzlich auch in den oben angegebenen Lösungsmitteln mit katalytischen Mengen von Mineralsäuren, wie Schwefelsäure, Salzsäure, Perchlorsäure oder p-Toluolsulfonsäure, durchgeführt werden.

Der überraschende Vorteil des erfindungsgemäßen Verfahrens liegt in der chemoselektiven Synthese von Imidazolderivaten unter Bildung nur eines Isomeren in einer einzigen Verfahrensstufe.

Für die sich gegebenenfalls anschließende Umesterung sind alle gebräuchlichen Methoden geeignet. Beispielsweise genannt seien die Umsetzung das Carbonsaureesters mit dem entsprechenden Alkohol in Gegenwart des Alkoholats oder mit dem entsprechenden Alkohol mit Titan-tetraalkoholat oder mit dem Alkohol in Gegenwart einer Säure. Die Umesterung wird bei Temperaturen von etwa 0 bis 120°C durchgeführt.

Die sich gegebenenfalls anschließende Verseifung der Estergruppe erfolgt zweckmäßigerweise alkalisch, wobei der Ester in verdünnter wäßriger Alkalilauge, wie Kalium- oder Natriumhydroxid, bis zur Rückflußtemperatur erhitzt wird.

Die Veresterung der Carboxylgruppe geschieht in an sich bekannter Weise mit dem entsprechenden Alkohol in Säure oder in Gegenwart eines aktivierten Säurederivats. Als aktivierte Säurederivate kommen zum Beispiel Säurechlorid, -imidazolid oder -anhydrid infrage.

Zur Amidierung wird die Imidazol-4-carbonsäure oder der entsprechende Ester mit Hilfe von N,N'-Carbonyldiimidazol oder Dicyclohexylcarbodiimid mit einem primären oder sekundären Amin der allgemeinen Formel

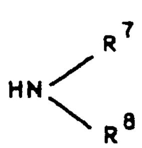

umgesetzt. Die Umsetzung kann auch in an sich bekannter Weise über aktivierte Säurederivate wie über das Anhydrid oder das gemischte Anhydrid mit Chlorameisensäureester erfolgen. Üblicherweise wird die Amidierung in einem aprotischen Lösungsmittel wie Dimethylformamid, Tetrahydrofuran, Toluol oder Methylenchlorid bei Temperaturen von etwa 0 bis 100°C vorgenommen.

Zur Einführung des 5-Oxadiazolylrestes kann auch die Imidazol-4-carbonsäure mit einem Amidoxim der Formel $R^9$-C(=NOH)$NH_2$, in der $R^9$ die in Formel I angegebene Bedeutung hat, in einem inerten Lösungsmittel bei Raumtemperatur bis zur Siedetemperatur des Reaktionsgemisches zur Kondensation gebracht werden. Als inerte Lösungsmittel sind beispielsweise Toluol und Dimethylformamid geeignet. Zweckmäßigerweise wird die freie Carbonsäure vor der Kondensationsreaktion in geeigneter Weise aktiviert. Hierzu kann die freie Säure in das gemischte Anhydrid, in den aktivierten Ester oder in das Chlorid überführt werden. Gut bewährt hat sich

eine Aktivierung mit Imidazol/Thionylchlorid in einem aprotischen Lösungsmittel wie Dioxan, Tetrahydrofuran, Dimethylformamid oder N-Methylpyrrolidon bei Temperaturen zwischen 0 und 50°C.

Die sich gegebenenfalls anschließende Abwandlung der Nitrilgruppe kann nach bekannten Methoden durchgeführt werden. Beispielsweise kann die Nitrilgruppe durch saure oder alkalische Hydrolyse in die Carbonylamid- oder Carboxylgruppe oder mit dem entsprechenden Alkohol unter Zugabe von Chlorwasserstoffgas über die Iminoestergruppe in die Estergruppe überführt werden.

Zur Einführung des 3-Oxadiazolylrestes wird das Imidazol-4-carbonitril in an sich bekannter Weise mit Hydroxylamin zum Amidoxim umgesetzt und anschließend mit einer Alkancarbonsäure der Formel $R^9$-COOH, in der $R^9$ die in Formel I angegebene Bedeutung hat, oder einem aktivierten Derivat der Säure in einem inerten Lösungsmittel kondensiert. Die Kondensation wird in der gleichen Weise wie bei der 5-Oxadiazolylverbindung durchgeführt.

Die als Ausgangsstoffe benutzten Aniline der allgemeinen Formel II und 2-Azabutadiene der allgemeinen Formel III sind überwiegend bekannt oder können nach bekannten Methoden hergestellt werden.

2-Azabutadiene sind zum Beispiel in Liebigs Ann. Chem. 1980, 344 und in DE-OS 29 19 891 beschrieben.

Die Herstellung der in den Beispielen eingesetzten neuen 2-Azabutadiene 2-4 wird anhand dar folgenden Beispiele erläutert :

Azabutadien 1 :

1.4-Bis (dimethylamino)-2-aza-1,3-butadien-3-cärbonsäure-ethylester gemäß Liebigs Ann. Chem. 1980, 344.

Azabutadien 2

1,4-Bis(dimethylamino)-3-(3-ethyl-1,2,4)-oxadiazol-5-yl)-2-aza-1,3-butadien

Eine Mischung von 11,5 g 5-Aminomethyl-3-ethyl-1,2,4-oxadiazol und 24 ml Dimethylformamiddimethylacetal wird 7 Stunden auf 80°C erhitzt ; dabei werden 10 ml entstandenes Methanol abdestilliert. Nach Zugabe von weiteren 12 ml DMF-acetal wird die Mischung 3 Stunden unter Rückfluß gekocht, dann fraktioniert destilliert. Die bei 155-160°C und 0,04 mbar (0,03 Torr) übergehende Fraktion (Azabutadien 2) wird in einer Ausbeute von 72% ($n_D^{20}$ 1. 5908) erhalten.

Die Herstellung der Aungangsverbindung erfolgt in nachstehender Weise :

a) 3-Ethyl-5-(phthalimidomethyl)-1,2,4-oxadiazol

Zu einer Lösung von 65,7 g Phthalimidoessigsäure in 500 ml Tetrahydrofuran (THF) (abs.) wird bei 40°C eine Suspension von 26,0 g Carbonyldiimidazol in 250 ml THF zugefügt. Nach ca. 1 Stunde ist keine Gasentwicklung mehr feststellbar. Jetzt wird eine Lösung von 28,2 g Propioamidoxim in 50 ml THF zugesetzt und 24 Stunden bei Raumtemperatur gerührt. Nach Abfiltrieren des Niederschlags wird das Filtrat im Vakuum eingeengt und nach Zusatz von 500 ml trockenem Xylol 6 Stunden am Wasserabscheider unter Rückfluß erhitzt. Die noch heiße Lösung wird vom öligen Rückstand getrennt und im Vakuum eingeengt. Nach Kristallisation aus Ethanol erhält man 31,5 g Phthalimid mit dem Schmelzpunkt 106-107°C

76,5% bezogen auf Carbonyldiimidazol).

b) 5-Aminomethyl-3-ethyl-1,2,4-oxadiazol

Eine Suspension von 32,2 g Phthalimid in 250 ml Methanol wird bei Raumtemperatur mit 4,5 g (140 mmol) Hydrazin versetzt, wobei sich die Substanz rasch auflöst. Das Reaktionsgemisch wird 3 Stunden unter Rückfluß gekocht, dann der gebildete Niederschlag abgesaugt, mit Methanol nachgewaschen und das Filtrat eingeengt. Nach Aufschlämmen des Rückstandes mit Diethylether wird erneut filtriert, eingeengt und das Öl am Kugelrohr destilliert : Siedepunkt 90-100°C, bei 0,04 mbar (0,03 Torr).

Ausbeute 14,87 g (91,6%) ; $n_D^{20}$ 1,4691.

Azabutadien 3 :

1,4-Bis(dimethylamino-3-cyano-2-azabutadien

Eine Mischung von 45 g Aminoacetonitril und 190 g Dimethylformamid-dimethylacetal wird zunächst unter Feuchtigkeitsausschluß 4 Stunden bei 100°C (Badtemperatur), dann 3 Stunden auf 120°C erhitzt. Dabei werden ca. 90 ml leichtflüchtiges Material (Methanol) abdestilliert, erneut 100 ml DMF-dimethylacetal zugefügt und weiter 5 Stunden auf 150°C (Badtemperatur) erhitzt. Nach der anschließenden fraktionierten Destillation im Vakuum werden erhalten :

Fraktion 1 (Siedepunkt 64-74°C/0,05 mbar (0,04 Torr) : 59,9 g (67,4%) Dimethylaminomethylenaminoacetonitril.

Fraktion 2 (Siedepunkt 115-125°C/0,04 mbar (0,03 Torr)) : 31 g (23,3%) der Titelverbindung.

Die Titelverbindung wird aus Hexan kristallisiert ;

Schmelzpunkt : 78-81°C.

Azabutadien 4 :

3-Cyano-1-dimethylamino-4-pyrrolidino-2-aza-1,3-pentadien

Eine Mischung von 22 g Dimethylaminomethylenaminoacetonitril, 27 g Dimethylacetamiddimethylacetal und 14 g Pyrrolidin wird 48 Stunden auf 80°C (Badtemperatur) erhitzt. Nach Einengen im Vakuum und anschließende Kugelrohrdestillation (160-185°C/0,01 mbar (0,08 Torr) werden 30 g der Titelverbindung erhalten, die aus Hexan kristallisiert wird.

Schmelzpunkt : 49-53°C (E.Z-Gemisch).

Azabutadien 5

1-Dimethylamino-3-(3-ethyl-1,2,4-oxadiazol-5-yl)-4-pyrrolidino-2-azapenta-1,3-dien (E.Z)

a) Eine Mischung aus 26 g 5-Aminomethyl-3-ethyl-1,2,4-oxadiazol und 30 ml Dimethylformamiddimethylacetal wird 6,5 h auf 80°C erhitzt.

Anschließend werden 16 ml Methanol abdestilliert und das gebildete Produkt wird durch Kugelrohrdestillation gereinigt. Man erhält 27,9 g (74,8%) 3-Ethyl-5-(H-dimethylaminomethylenaminomethyl)-1,2,4-oxadiazol.

Siedepunkt : 130-150°C/0,06 mbar (0,05 Torr) ; $n_D^{20}$ : 1,4924.

b) 13,6 g des unter a erhaltenen Produktes, 15,0 g Dimethylacetamiddimethylacetal und 8,0 g Pyrrolidin werden 21 h unter Stickstoff auf 80°C erhitzt. Anschließend wird der gebildete Alkohol abdestilliert und das Reaktionsprodukt durch Kugelrohrdistillation gereinigt. Man erhält 15,4 g einer Fraktion, die bei 215-230°C/0,05 mbar (0,004 Torr) übergeht. Durch Umkristallisation aus n-Hexan erhält man 9,5 g (45,7%) der Titelverbindung mit einem Schmelzpunkt von 59-62°C.

Azabutadien 6

1.Dimethylamino-3-(3-ethyl-1,2,4-oxadiazol-5-yl)-5-methoxy-4-(1-pyrrolidinyl-2-azapenta-1,3-dien (E.Z)

a) Methoxyessigsäure-dimethylamid-dimethylacetal

Zu 53,4 g Trimethyloxonium-tetrafluorborat werden unter Kühlung 42,2 g Methoxyessigsaure-dimethylamid in drei Portionen gegeben. Das Reaktionsgemisch wird anschließend 2 h bei Raumtemperatur gerührt und danach über Nacht stehengelassen. Nach dem Lösen in 40 ml Dichlormethan wird das gebildete Salz langsam zu einer Lösung von Natriummethoxid in Methanol (hergestellt durch Auflösen von 10,4 g Natrium in 225 ml Methanol gegeben. Danach wird 2 h bei Raumtemperatur gerührt. Zur Aufarbeitung wird der gebildete Niedershlag abgesaugt und mit wenig Ethanol ausgewaschen. Das Filtrat bildet nach dem Abdestillieren der Lösungsmittel 2 Phasen. Durch Kugelrohrdestillation erhalt man aus der oberen Phase 9.08 g (15%) des gewünschten Produktes.

Siedepunkt : 54-57°C/18,7 mbar (14 Torr) $n_D^{20}$ : 1,4204

b) 8,8 g des unter a) dargestellten Produktes werden mit 6,6 g 3-Ethyl-5-(N-dimethylaminomethylenaminomethyl)-1,2,4-oxadiazol und 4,5 ml Pyrrolidin analog der Herstellung von Azabutadien 5 umgesetzt. Durch Kugelrohrdestillation erhalt man 11,8 g (11%) Azabutadien 6.

Siedepunkt : 200-240°C/0,06 mbar (0,05 Torr)

Beispiel 1

a) 1-(3-Chlorphenyl)-imidazol-4-carbonsaüre-ethylester

Unter Kühlung werden 2,2 g Azabutadien 1 in 8 ml Eisessig getropft und anschließend mit 1 g 3-Cloranilin versetzt. Die Mischung wird zunächst 30 Minuten bei Raumtemperatur gerührt, anschließend 5 Stunden auf 80°C (Badtemperatur) erhitzt. Nach Aufarbeitung mit Natriumhydrogencarbonatlösung wird der kristalline Rückstand aus Diisopropylether umkristallisiert. Man erhält 1,63 g der Titelverbindung (81% bezogen auf Chloranilin),

Schmelzpunkt : 104-105°C.

Analog Beispiel 1 a) werden erhalten :

b) 1-(3-,4-Dichlorphenyl)-imidazol-4-carbonsäure-ethylester aus 3,4-Dichloranilin

c) 1-(4-Chlorphenyl)-imidazol-4-carbonsäure-ethylester aus 4-Chloranilin

d) 1-(4-Fluorphenyl)-imidazol-4-carbonsäure-ethylester aus 4-Fluoranilin.

Beispiel 2

a) 1-(3-Chlorphenyl)-imidazol-4-carbonsäure-isopropylester

Eine Lösung von 0,4 g 1-(3-Chlorphenyl)-imidazol-4-carbonsäure-ethylester in 40 ml i-Propanol wird mit 0,5 ml Titantetraisopropanolat versetzt und 8 Stunden unter Rückfluß gekocht. Nach Einengen der Lösung wird über Kieselgel filtriert und aus Diisopropylether umkristallisiert. Man erhält 267 mg (63%) der Titelverbindung vom Schmelzpunkt 109°C aus Diisopropylether.

In analoger Weise wird hergestellt :

b) 1-(4-Fluorphenyl)-imidazol-4-carbonsäure-isopropylester

Beispiel 3

a) 1-(3-Chlorphenyl)-imidazol-4-carbonsäure

Eine Suspension von 1 g 1-(3-Chlorphenyl)-imidazol-4-carbonsäure-ethylester in 10 ml 2 N KOH wird 2 Stunden auf 110°C (Badtemperatur) erhitzt. Nach dem Abkühlen wird auf pH 3-4 angesäuert mit 4 N HCl und der Niederschlag aus i-Propanol umkristallisiert. Man erhält 0,73 g (82%) mit Schmelzpunkt 195°C.

In analoger Weise wird erhalten :

b) 1-(4-Fluorphenyl)imidazol-4-carbonsäure

Beispiel 4

a) 1-(3-Chlorphenyl)-imidazol-4-carbonsäure-diethylamid

In eine Lösung von 0,56 g Säure (Beispiel 3 a) in 10 ml Dimethylformamid werden 0.5 g N,N'-Carbonyldiimidazol in 5 ml DMF eingetropft, anschließend die Lösung 30 Minuten auf 60°C erhitzt bis zur beendeten Gasentwicklung und dann mit 1,3 ml Diethylamin versetzt. Die Mischung wird 2 Stunden auf 90°C erhitzt, dann im Vakuum eingeengt und mit Wasser gefällt. Das Rohprodukt wird aus Diisopropylether kristallisiert. Man erhält 0.48 g (70%) der Titelverbindung vom Schmelzpunkt 105°C aus Diisopropylether.

In analoger Weise wird erhalten :

b) 1-(4-Fluorphenyl)-imidazol-4-carbonsäure-diisopropylamid

Beispiel 5

a) 1-(3-Chlorphenyl)-4-(3-ethyl-1,2,4-oxadiazol-5-yl)-imidazol

Die Herstellung erfolgt aus 3-Chloranilin und Azabutadien 2 analog Beispiel 1a). Man erhalt 60% vom Schmelzpunkt 134-135°C (Ethylacetat).

In analoger Weise werden aus den entsprechenden Anilinen erhalten :

b) 1-(3,4-Dichlorphenyl)-4-(3-ethyl-1,2,4-oxadiazol-5-yl)-imidazol

c) 1-(4-Chlorphenyl)-4-(3-ethyl-1,2,4-oxadiazol-5-yl)-imidazol

d) 1-(4-Fluorphenyl)-4-(3-ethyl-1,2,4-oxadiazol-5-yl)imidazol

e)1-(3-Bromphenyl)-4-(3-ethyl-1,2,4-oxadiazol-5-yl)imidazol

Beispiel 6

a) 1-(3-Chlorphenyl)-imidazol-4-carbonitril

Die Herstellung erfolgt aus 3-Chloranilin und Azabutadien 3 analog Beispiel 1 a). Man erhält neben 25% des 1-(3-Chlor-phenyl)-imidazol-4-carbonsäureamids nach Chromatographie 35% der Titelverbindung.

Beispiel 7

a) 1-(3-Chlorphenyl)-5-methyl-imidiazol-4-carbonitril

Die Herstellung erfolgt aus 3-Chloranilin und Azabutadien 4 analog Beispiel 1 a). Nach Chromatographie an Kieselgel erhält man 30% der Titelverbindung.

Schmelzpunkt : 117-118°C aus Diisopropylether und analog 7a als Nebenprodukt

b) 1-(3-Chlorphenyl)-5-methyl-imidazol-4-carbonsäureamid

Schmelzpunkt : 179-181°C (Essigester) mit 3,4% Ausbeute

In analoger Weise wird erhalten :

c) 1-(4-Fluorphenyl)-5-methyl-imidazol-4-carbonitril.

Beispiel 8

a) 1-(3-Chlorphenyl)-4-(5-ethyl-1,2,4-oxadiazol-3-yl)-5-methyl-imidazol

653 mg 1-(3-Chlorphenyl)-5-methyl-imidazol-4-carbonitril aus Beispiel 7a), 695 mg Hydroxylammoniumchlorid und 690 mg Kaliumcarbonat werden in 32 ml Ethanol 1,5 h auf 110°C erhitzt. Anschließend wird eingeengt, der Rückstand mit Wasser verrührt und der gebildete Feststoff abgesaugt. Nach dem Trocknen wird aus Ethanol umkristallisiert.

Ausbeute : 600 mg (79,8%) 1-(3-Chlorphenyl)-5-methyl-imidazol-4-formamidoxim vom Schmelzpunkt 206-209°C.

b) 577 mg des unter 8a) erhaltenen Formamidoxims werden in 40 ml wasserfreiem Tetrahydrofuran vorgelegt und vorsichtig mit 283 mg Propionylchlorid versetzt. Anschließend wird 16 h bei Raumtemperatur gerührt. Nachdem das Tetrahydrofuran abdestilliert worden ist, wird der Rückstand in 25 ml Xylol aufgenommen

und der Ansatz 3,5 h refluxiert. Nach dem Einengen und Chromatographie des Rohproduktes an Kieselgel mit Toluol/Essigester (1 :1) erhält man 0,74 g 1-(3-Chlorphenyl)-4-(5-ethyl-1,2,4-oxadiazol-3-yl)-5-methyl-imidazol.

Schmelzpunkt : 71-72°C (Diisopropylether).

Beispiel 9

1-(3-Chlorphenyl)-4-(3-ethyl-1,2,4-oxadiazol-5-yl)-5-methyl-imidazol

Die Herstellung erfolgt aus 3-Chloranilin und Azabutadien 5 analog Biespiel1a). Nach Chromatographie an Kieselgel erhält man 31% der Titelverbindung.

Schmelzpunkt : 96-97°C (Diisopropylether).

Beispiel 10

1-(3-Chlorphenyl)-4-(3-ethyl-1,2,4-oxadiazol-5-yl)-5-methoxymethyl-imidazol

Die Herstellung erfolgt aus 3-Chloranilin und Azabutadien 6 analog Beispiel 1a). Nach Chromatographie an Kieselgel erhalt man 46% der Titelverbindung.

Schmelzpunkt : 125-126°C (Diisopropylether/Ethanol).

## Ansprüche

1. Imidazolderivate der allgemeinen Formel I

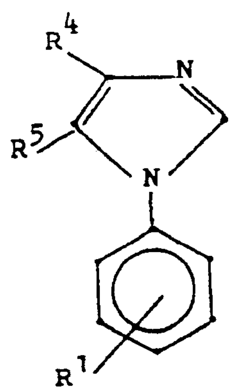

(I),

worin

$R^1$ Wasserstoff oder Halogen in o-, m- oder p-Stellung, wobei das Halogen einfach oder mehrfach am Phenylrest auftreten kann,

$R^4$ $-COOR^6$, 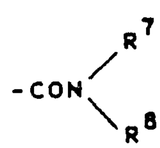 , $-CN$,

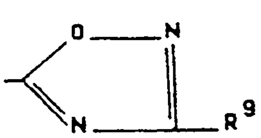 oder 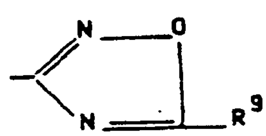

mit $R^6$ und $R^9$ in der Bedeutung von Wasserstoff oder einer geraden oder verzweigten Alkylgruppe mit 1 bis 6 Kohlenstoffatomen, $R^7$ und $R^8$ sind gleich oder verschieden und stellen Wasserstoff oder eine gerade oder verzweigte Alkylgruppe mit 1 bis 6 Kohlenstoffatomen dar oder $R^7$ und $R^8$ stellen gemeinsam mit dem Stickstoffatom einen gegebenenfalls ein weiteres Heteroatom enthaltenden gesättigten heterocyclischen Fünf- oder Sechsring dar und

$R^5$ Wasserstoff, eine Alkylgruppe mit 1 bis 6 Kohlenstoffatomen oder eine Alkoxyalkylgruppe mit 1 bis 6 Kohlenstoffatomen bedeuten.

2. 1-(3-Chlorphenyl)-imidazol-4-carbonsäure-ethylester,
1-(3,4-Dichlorphenyl)-imidazol-4-carbonsäure-ethylester,
1-(4-Chlorphenyl)-imidazol-4-carbonsäure-ethylester,
1-(4-Fluorphenyl)-imidazol-4-carbonsäure-ethylester,
1-(3-Chlorphenyl)-imidazol-4-carbonsäure-isopropylester,
1-(4-Fluorphenyl)-imidazol-4-carbonsäure-isopropylester,
1-(3-Chlorphenyl)-imidazol-4-carbonsäure und
1-(4-Fluorphenyl)-imidazol-4-carbonsäure nach Anspruch 1.

3. 1-(3-Chlorphenyl)-imidazol-4-carbonsäure-diethylamid
1-(4-Fluorphenyl)-imidazol-4-carbonsäure-diisopropylamid.
1-(3-Chlorphenyl)-5-methyl-imidazol-4-carbonsäureamid nach Anspruch 1.

4. 1-(3-Chlorphenyl)-4-(3-ethyl-1,2,4-oxadiazol-5-yl)-imidazol,
1-(3,4-Dichlorphenyl)-4-(3-ethyl-1,2,4-oxadiazol-5-yl)-imidazol,
1-(4-Chlorphenyl)-4-(3-ethyl-1,2,4-oxadiazol-5-yl)-imidazol,
1-(4-Fluorphenyl)-4-(3-ethyl-1,2,4-oxadiazol-5-yl)-imidazol
1-(3-Bromphenyl)-4-(3-ethyl-1,2,4-oxadiazol-5-yl)-imidazol.
1-(3-Chlorphenyl)-4-(3-ethyl-1,2,4-oxadiazol-5-yl)-5-methyl-imidazol.
1-(3-Chlorphenyl)-4-(5-ethyl-1,2,4-oxadiazol-5-yl)-5-methyl-imidazol.
1-(3-Chlorphenyl)-4-(3-ethyl-1,2,4-oxadiazol-5-yl)-5-methoxy-methyl-imidazol nach Anspruch 1.

5. 1-(3-Chlorphenyl)-imidazol- 4-carbonitril,
1-(3-Chlorphenyl)-5-methyl-imidazol-4-carbonitril und
1-(4-Fluorphenyl)-5-methyl-imidazol-4-carbonitril nach Anspruch 1.

6. Arzneimittel auf Basis der Verbindungen gemäß den Ansprüchen 1 bis 5.

7. Verfahren zur Herstellung von Imidazolderivaten der allgemeinen Formel I

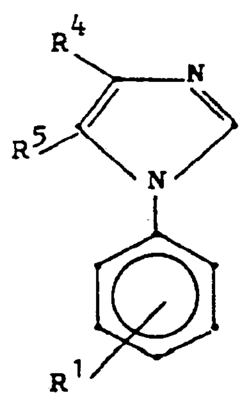

(I),

worin

$R^1$ Wasserstoff oder Halogen in o-, m- oder p-Stellung, wobei das Halogen einfach oder mehrfach am Phenylrest auftreten kann

$R^4$ $-COOR^6$, 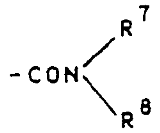 , $-CN$,

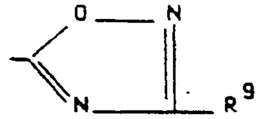 oder 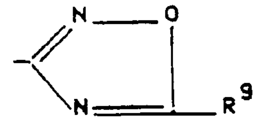

mit $R^6$ und $R^9$ in der Bedeutung von Wasserstoff oder einer geraden oder verzweigten Alkylgruppe mit 1 bis 6 Kohlenstoffatomen, $R^7$ und $R^8$ sind gleich oder verschieden und stellen Wasserstoff oder eine gerade oder verzweigte Alkylgruppe mit 1 bis 6 Kohlenstoffatomen dar oder $R^7$ und $R^8$ stellen gemeinsam mit dem Stickstoffatom einen gegebenenfalls ein weiteres Heteroatom enthaltenden gesättigten heterocyclischen Fünf- oder Sechsring dar und

$R^5$ Wasserstoff, eine Alkylgruppe mit 1 bis 6 Kohlenstoffatomen oder eine Alkoxyalkylgruppe mit 1 bis 6 Kohlenstoffatomen bedeuten.

dadurch gekennzeichnet, daß man ein Anilin der allgemeinen Formel II

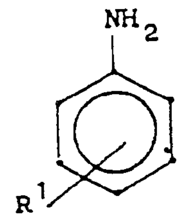

(II),

worin

$R^1$ die in Formel I angegebene Bedeutung hat, mit einem 2-Azabutadien der allgemeinen Formel III

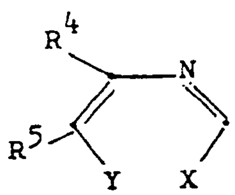

(III),

worin

$R^4$ und $R^5$ die in Formel I angegebene Bedeutung haben und

X und Y Fluchtgruppen darstellen, in Gegenwart von Säuren bei Temperaturen von 0 bis 150°C umsetzt und gegebenenfalls eine im Molekül vorhandene Estergruppe umestert oder verseift, eine freie Carboxylgruppe gegebenenfalls verestert, amidiert oder mit einem Amidoxim der Formel $R^9$-C(=NOH)$HH_2$ zum 5-Oxadiazolylderivat umsetzt und gegebenenfalls eine im Molekül vorhandene Nitrilgruppe zur Carbonylamid- oder Carboxylgruppe hydrolisiert oder über die Iminoestergruppe in die Estergruppe ($COOR^6$) oder mit Hydroxylamin über das Amodoxim und anschließend mit einer Alkancarbonsäure der Formel $R^9$-COOH oder einem aktivierten Derivat der Säure in das 3-Oxadiazolylderivat überführt.

## Claims

1. Imidazole derivatives of general formula I

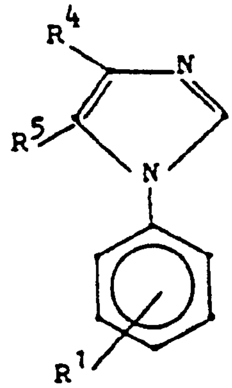

(I)

wherein

$R^1$ represents hydrogen or halogen in the o-, m- or p-position, and the halogen can occur one or more times in the phenyl radical,

$R^4$ represents

$-COOR^6$, 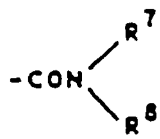 , $-CN$,

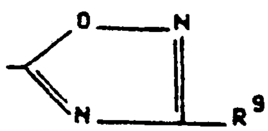 or 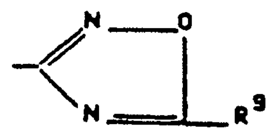

with $R^6$ and $R^9$ representing hydrogen or a straight or branched alkyl group with 1 to 6 carbon atoms, $R^7$ and $R^8$ are the same or different and represent hydrogen or a straight or branched alkyl group with 1 to 6 carbon atoms or $R^7$ and $R^8$ together with the nitrogen atom represent a saturated heterocyclic five-membered or six-membered ring optionally containing a further hetero atom, and

$R^5$ represents hydrogen, an alkyl group with 1 to 6 carbon atoms or an alkoxyalkyl group with 1 to 6 carbon atoms.

2. 1-(3-chlorophenyl)-imidazole-4-carboxylic acid ethyl ester,
1-(3,4-dichlorophenyl)-imidazole-4-carboxylic acid ethyl ester,
1-(4-chlorophenyl)-imidazole-4-carboxylic acid ethyl ester,
1-(4-fluorophenyl)-imidazole-4-carboxylic acid ethyl ester,
1-(3-chlorophenyl)-imidazole-4-carboxylic acid isopropyl ester,
1-(4-fluorophenyl)-imidazole-4-carboxylic acid isopropyl ester,
1-(3-chlorophenyl)-imidazole-4-carboxylic acid and
1-(4-fluorophenyl)-imidazole-4-carboxylic acid according to claim 1.

3. 1-(3-chlorophenyl)-imidazole-4-carboxylic acid diethylamide,
1-(4-fluorophenyl)-imidazole-4-carboxylic acid diisopropylamide,
1-(3-chlorophenyl)-5-methyl-imidazole-4-carboxylic acid amide according to claim 1.

4. 1-(3-chlorophenyl)-4-(3-ethyl-1,2,4-oxadiazol-5-yl)-imidazole,
1-(3,4-dichlorophenyl)-4-(3-ethyl-1,2,4-oxadiazol-5-yl)-imidazole,
1-(4-chlorophenyl)-4-(3-ethyl-1,2,4-oxadiazol-5-yl)-imidazole,
1-(4-fluorophenyl)-4-(3-ethyl-1,2,4-cxadiazol-5-yl)-imidazole,
1-(3-bromophenyl)-4-(3-ethyl-1,2,4-oxadiazol-5-yl)-imidazole,
1-(3-chlorophenyl)-4-(3-ethyl-1,2,4-oxadiazol-5-yl)-5-methyl-imidazole,
1-(3-chlorophenyl)-4-(5-ethyl-1,2,4-oxadiazol-3-yl)-5-methyl-imidazole,

1-(3-chlorophenyl)-4-(3-ethyl-1,2,4-oxadiazol-5-yl)-5-methoxymethyl-imidazole according to claim 1.

5. 1-(3-chlorophenyl)-imidazole-4-carbonitrile,
1-(3-chlorophenyl)-5-methyl-imidazole-4-carbonitrile and
1-(4-fluorophenyl)-5-methyl-imidazole-4-carbonitrile according to claim 1.

6. Pharmaceutical based on the compounds according to claims 1 to 5.

7. Process for the manufacture of imidazole derivatives of general formula I

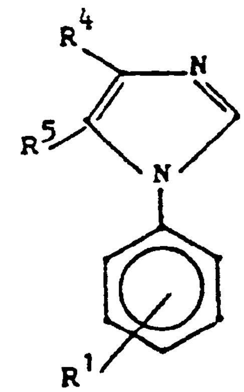

(I)

wherein

$R^1$ represents hydrogen or halogen in the o-, m- or p-position, and the halogen can occur one or more times in the phenyl radical,

$R^4$ represents

$-COOR^6$, 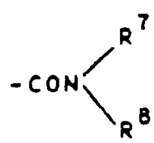 . $-CN$,

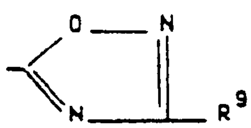 or 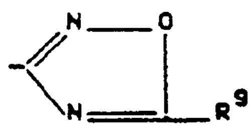

with $R^6$ and $R^9$ representing hydrogen or a straight or branched alkyl group with 1 to 6 carbon atoms, $R^7$ and $R^8$ are the same or different and represent hydrogen or a straight or branched alkyl group with 1 to 6 carbon atoms or $R^7$ and $R^8$ together with the nitrogen atom represent a saturated heterocyclic five-membered or six-membered ring optionally containing a further hetero atom, and

$R^5$ represents hydrogen, an alkyl group with 1 to 6 carbon atoms or an alkoxyalkyl group with 1 to 6 carbon atoms,

characterised in that an aniline of general formula II

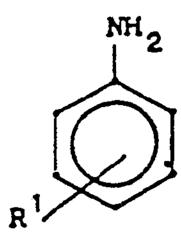

(II),

wherein

$R^1$ has the meaning indicated in formula I, is reacted with a 2-azabutadiene of general formula III

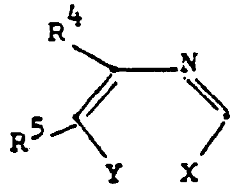

(III),

wherein

$R^4$ and $R^5$ have the meaning indicated in formula I and X and Y represent leaving groups, in the presence of acids, at temperatures of from 0 to 150°C, and optionally an ester group present in the molecule is transesterified or hydrolysed, a free carboxyl group optionally is esterified, amidated or reacted with an amidoxime of formula $R^9$-C(=NOH)$NH_2$ to form the 5-oxadiazolyl derivative, and optionally a nitrile group present in the molecule is hydrolysed to the carbonyl amide or carboxyl group or converted via the imino ester group into the ester group ($COOR^6$) or with hydroxylamine via the amidoxime and then with an alkanecarboxylic acid of formula $R^9$-COOH or an activated derivative of the acid into the 3-oxadiazolyl derivative.

## Revendications

1. Dérivés de l'imidazole qui répondent à la formule générale I :

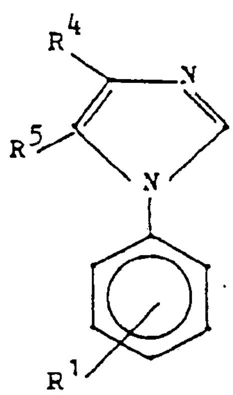

(I),

dans laquelle :

$R^1$ représente l'hydrogène ou un halogène en position o, m ou p, l'halogène pouvant être unique ou en plusieurs exemplaires sur le radical phényle,

$R^4$ représente l'un des radicaux suivants :

$-COOR^6$,

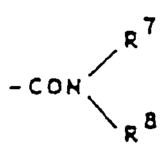

,

-CN,

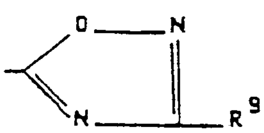

et

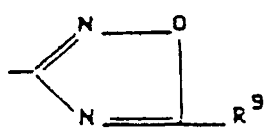

dans lesquels $R^6$ et $R^9$ représentent chacun l'hydrogène ou un alkyle linéaire ou ramifié qui contient de 1 à 6 atomes de carbone, $R^7$ et $R^8$ représentent chacun, indépendamment l'un de l'autre, l'hydrogène ou un alkyle linéaire ou ramifié qui contient de 1 à 6 atomes de carbone, ou $R^7$ et $R^8$ forment ensemble et avec l'atome d'azote un hétérocycle saturé à 5 ou 6 maillons qui contient éventuellement un hétéroatome supplémentaire, et

$R^5$ représente l'hydrogène, un alkyle contenant de 1 à 6 atomes de carbone ou un alcoxy-alkyle contenant de 1 à 6 atomes de carbone.

2. Composés selon la revendication 1, en l'espèce :
le (chloro-3 phényl)-1 imidazole-carboxylate-4 d'éthyle,
le (dichloro-3,4 phényl)-1 imidazole-carboxylate-4 d'éthyle,
le (chloro-4 phényl)-1 imidazole-carboxylate-4 d'éthyle,
le (fluoro-4 phényl)-1 imidazole-carboxylate-4 d'éthyle,
le (chloro-3 phényl)-1 imidazole-carboxylate-4 d'isopropyle,
le (fluoro-4 phényl)-1 imidazole-carboxylate-4 d'isopropyle,
l'acide (chloro-3 phényl)-1 imidazole-carboxylique-4 et
l'acide (fluoro-4 phényl)-1 imidazole-carboxylique-4

3. Composés selon la revendication 1, en l'espèce :
le diéthylamide de l'acide (chloro-3 phényl)-1 imidazole-carboxylique-4,
le diisopropylamide de l'acide (fluoro-4 phényl)-1 imidazole-carboxylique-4 et
le (chloro-3 phényl)-1 méthyl-5 imidazole-carboxamide-4.

4. Composés selon la revendication 1, en l'espèce :
le (chloro-3 phényl)-1 (éthyl-3 oxadiazole-1,2,4 yl-5)-4 imidazole,
le (dichloro-3,4 phényl)-1 (éthyl-3 oxadiazole-1,2,4 yl-5)-4 imidazole,
le (chloro-4 phényl)-1 (éthyl-3 oxadiazole-1,2,4 yl-5)-4 imidazole,
le (fluoro-4 phényl)-1 (éthyl-3 oxadiazole-1,2,4 yl-5)-4 imidazole,
le (bromo-3 phényl)-1 (éthyl-3 oxadiazole-1,2,4 yl-5)-4 imidazole,
le (chloro-3 phényl)-1 (éthyl-3 oxadiazole-1,2,4 yl-5)-4 méthyl-5 imidazole,
le (chloro-3 phényl)-1 (éthyl-5 oxadiazole-1,2,4 yl-3)-4 méthyl-5 imidazole et
le (chloro-3 phényl)-1 (éthyl-3 oxadiazole-1,2,4 yl-5)-4 méthoxyméthyl-5 imidazole.

5. Composés selon la revendication 1, en l'espèce :
le (chloro-3 phényl)-1 imidazole-carbonitrile-4,
le (chloro-3 phényl)-1 méthyl-5 imidazole-carbonitrile-4 et
le (fluoro-4 phényl)-1 méthyl-5 imidazole-carbonitrile-4.

6. Médicament à base de composés selon l'une quelconque des revendications 1 à 5.

7. Procédé pour préparer des dérivés de l'imidazole répondant à la formule générale I :

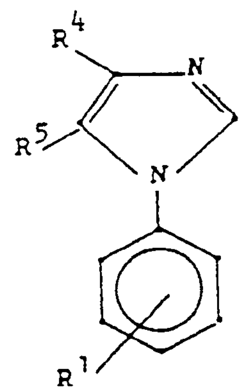

(I),

dans laquelle :

$R^1$ représente l'hydrogène ou un halogène en position o, m ou p, l'halogène pouvant être unique ou en plusieurs exemplaires sur le radical phényle,

$R^4$ représente l'un des radicaux suivants :

$-COOR^6$, 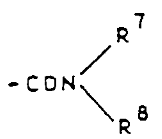 , $-CN$,

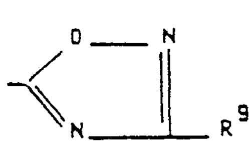 et 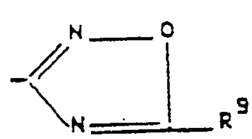

dans lesquels $R^6$ et $R^9$ représentent chacun l'hydrogène ou un alkyle linéaire ou ramifié qui contient de 1 à 6 atomes de carbone, $R^7$ et $R^8$ représentent chacun, indépendamment l'un de l'autre, l'hydrogène ou un alkyle linéaire ou ramifié qui contient de 1 à 6 atomes de carbone, ou $R^7$ et $R^8$ forment ensemble et avec l'atome d'azote un hétérocycle saturé à 5 ou 6 maillons qui contient éventuellement un hétéroatome supplémentaire, et

$R^5$ représente l'hydrogène, un alkyle contenant de 1 à 6 atomes de carbone ou un alcoxy-alkyle contenant de 1 à 6 atomes de carbone, procédé caractérisé en ce qu'on fait réagir une aniline répondant à la formule générale II :

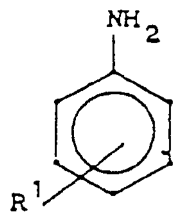

(II),

dans laquelle $R^1$ a la signification qui lui a été donnée à propos de la formule I, avec un aza-2 butadiène répondant à la formule générale III :

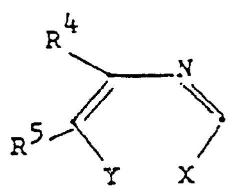

(III),

dans laquelle $R^4$ et $R^5$ ont les significations indiquées à propos de la formule I, et X et Y représentent des radicaux éliminables, en présence d'acides, à des températures de 0 à 150°C, éventuellement on transestérifie ou saponifie un radical d'ester présent dans la molécule, éventuellement on estérifie ou amidifie un radical carboxy libre ou on le transforme, par réaction avec une carboxamide-oxime de formule $R^9$-C(=NOH)$NH_2$, en le dérivé oxadiazolique-5, et éventuellement on hydrolyse un radical cyano présent dans la molécule pour le convertir en un radical carbamoyle ou carboxy ou on le transforme, en passant par l'ester carboximidique, en le radical d'ester ($COOR^6$) ou, par réaction avec l'hydoxylamine, en passant par la carboxamide-oxime, puis réaction avec un acide alcanoïque de formule $R^9$-COOH ou un dérivé activé d'un tel acide, en le dérivé oxadiazolylique-3.